# EUROPÄISCHE PATENTSCHRIFT

(11) **EP 2 550 979 B1**
(45) Veröffentlichungstag und Bekanntmachung des Hinweises auf die Patenterteilung: **07.06.2017**
(21) Anmeldenummer: 12005191.7
(22) Anmeldetag: 13.07.2012
(51) Int. Cl.: A61L 24/04, A61L 24/06, A61L 27/16, A61L 27/26

(54) **Kit und Verfahren zur Herstellung von Knochenzement**
Kit and method for producing bone cement
Kit et procédé de fabrication de ciment osseux

(30) Priorität: 27.07.2011 DE 102011108574; 15.08.2011 US 201161523557 P
(43) Veröffentlichungstag der Anmeldung: 30.01.2013
(73) Patentinhaber: Heraeus Medical GmbH, 61273 Wehrheim (DE)
(72) Erfinder: Vogt, Sebastian, Dr., 99092 Erfurt (DE); Büchner, Hubert, Dr., 90491 Nürnberg (DE)
(74) Vertreter: Heraeus IP

(56) Entgegenhaltungen:
- EP-A2- 2 052 748

## Beschreibung

Die vorliegende Erfindung betrifft einen Kit zur Herstellung von Knochenzement und ein Verfahren zur Herstellung von Knochenzement.

Konventionelle Poly(methylmethacrylat)-Knochenzemente (PMMA-Knochenzemente) sind seit Jahrzehnten bekannt und gehen auf die grundlegenden Arbeiten von Sir Chamley zurück (Charnley, J.: Anchorage of the femoral head prosthesis of the shaft of the femur. J. Bone Joint Surg. 42 (1960) 28-30). Der Grundaufbau der PMMA-Knochenzemente ist seitdem prinzipiell gleich geblieben. PMMA-Knochenzemente bestehen aus einer flüssigen Monomerkomponente und einer Pulverkomponente. Die Monomerkomponente enthält im Allgemeinen (i) das Monomer Methylmethacrylat und (ii) einen darin gelösten Aktivator (zum Beispiel N,N-Dimethyl-p-toluidin). Die Pulverkomponente umfasst (i) ein oder mehrere Polymere, die auf Basis von Methylmethacrylat und Comonomeren, wie Styrol, Methylacrylat oder ähnlichen Monomeren, durch Polymerisation, vorzugsweise Suspensionspolymerisation, hergestellt sind, (ii) einen Röntgenopaker und (iii) einen Initiator (zum Beispiel Dibenzoylperoxid). Beim Vermischen der Pulverkomponente mit der Monomerkomponente entsteht durch Quellung der Polymere der Pulverkomponente im Methylmethacrylat der Monomerkomponente ein plastisch verformbarer Teig. Gleichzeitig reagiert der Aktivator N,N-Dimethyl-p-toluidin mit Dibenzoylperoxid, das unter Bildung von Radikalen zerfällt. Die gebildeten Radikale initiieren die radikalische Polymerisation des Methylmethacrylates. Mit fortschreitender Polymerisation des Methylmethacrylates erhöht sich die Viskosität des Zementteigs bis der Zementteig erstarrt und damit ausgehärtet ist.

Die grundlegenden mechanischen Anforderungen an PMMA-Knochenzemente, wie 4-Biegefestigkeit, Biegemodul und Druckfestigkeit, sind in der ISO 6833 beschrieben. Für den Anwender der PMMA-Knochenzemente ist die Eigenschaft der Klebfreiheit des PMMA-Knochenzements von wesentlicher Bedeutung. Der Begriff Klebfreiheit ist in der ISO5833 definiert und zeigt an, dass der PMMA-Knochenzement nach dem Vermischen der Komponenten durch Quellung der im Zementpulver enthaltenen Polymere im Monomer die Verarbeitungsphase erreicht hat. Grundsätzlich muss ein PMMA-Knochenzement klebfrei sein, damit der Anwender den Zement formen und applizieren kann. Der PMMA-Knochenzement darf nicht an den Handschuhen und an Applikationshilfen wie Mischsystemen, Tiegeln oder Spateln kleben.

Der wesentliche Nachteil der bisherigen PMMA-Knochenzemente für den medizinischen Anwender besteht darin, dass der Anwender die flüssige Monomerkomponente mit der Pulverkomponente unmittelbar vor der Zementapplikation in einem Mischsystem oder in Tiegeln vermischen muss. Dabei können leicht Mischungsfehler auftreten, die die Zementqualität negativ beeinflussen. Ferner muss das Vermischen der Komponenten zügig erfolgen. Dabei kommt es darauf an, dass das gesamte Zementpulver ohne Klumpenbildung mit der Monomerkomponente vermischt wird und dass beim Vermtschungsvorgang der Eintrag von Luftblasen vermieden wird. Bei der Verwendung von Vakuummischsystemen wird im Gegensatz zur Handanmischung die Bildung von Luftblasen im Zementteig weitgehend verhindert. Beispiele für Mischsysteme sind in den Schriften US 4015945 A, EP 0674888 A1 und JP 2003181270 A offenbart. Vakuummischsysteme machen jedoch eine zusätzliche Vakuumpumpe erforderlich und sind daher relativ kostenintensiv. Nach der Vermischung der Monomerkomponente mit der Pulverkomponente muss ferner je nach Zementtyp eine gewisse Zeit gewartet werden, bis der Zementteig klebfrei ist und appliziert werden kann. Aufgrund der vielen Fehlermöglichkeiten beim Anmischen von konventionellen PMMA-Knochenzementen wird zudem entsprechend geschultes Personal benötigt. Die entsprechenden Schulungen sind mit einem erheblichen Kostenaufwand verbunden. Weiterhin kommt es bei der Vermischung der flüssigen Monomerkomponente mit der Pulverkomponente zu einer Belastung der Anwender durch Monomerdämpfe und durch Freisetzung von pulverförmigen Zementpartikeln. Ein weiterer wesentlicher Nachteil der konventionellen PMMA-Knochenzemente besteht darin, dass sowohl die Pulverkomponente als auch die Monomerkomponente jeweils doppelt steril verpackt hergestellt werden müssen, was wenigstens vier sterile Packmittel für eine Knochenzementpackung erfordert.

Als Alternative zu den konventionellen Pulver-Flüssigkeits-Polymethylmethacrylatknochenzementen wird im Patent DE 102007050762 B3 ein Knochenzement vorgeschlagen, der zwei Pasten umfasst. Diese Pasten enthalten jeweils ein radikalisch polymerisierbares Methacrylatmonomer, ein in diesem Methacrylatmonomer lösliches Polymer und ein in diesem Methacrylatmonomer unlösliches partikuläres Polymer. In einer dieser Pasten ist zudem ein radikalischer Polymerisationsinitiator enthalten, während die andere Paste einen Polymerisationsaktivator aufweist. Der aus diesen Pasten hergestellte Knochenzement weist als Folge der gewählten Zusammensetzung eine ausreichend hohe Viskosität und Kohäsion auf, um dem Blutungsdruck bis zur Aushärtung standzuhalten. Beim Vermischen der beiden Pasten reagiert der Polymerisationsinftiator mit dem Akzelerator unter Bildung von Radikalen, die die radikalische Polymerisation der Methacrylatmonomere initiieren. Durch die fortschreitende Polymerisation härtet die Paste unter Verbrauch der Methacrylatmonomere aus. Es wurde gefunden, dass auch dann, wenn als im Methacrylatmonomer unlösliches partikuläres Polymer hochvernetzte Poly(methylmethacrylat)-Partikei verwendet werden, diese geringe Anteile an Methacrylatmonomer und darin gelösten Verbindungert aufnehmen und einschließen. Dadurch enthalten die unlöslichen Polymerpartikel der einen Paste Einschlüsse aus Monomerflüssigkeit und darin gelöstem Initiator, während die unlöslichen Polymerpartikel der anderen Paste ihrerseits Einschlüsse aus Monomerflüssigkeit und darin gelöstem Akzelerator enthalten. Nach dem Vermischen der beiden Pasten härtet die aus dem Methacrylatmonomer und dem gelösten Polymer bestehende Phase, in der die unlöslichen Polymerpartikel suspendiert sind, unter Herstellung von applikationsfähigem Knochenzement aus. In der Folgezeit diffundiert die zunächst eingeschlossene Monomerflüssigkeit aus den unlöslichen Polymerpartikeln heraus und polymerisiert nach. Die aus den unlöslichen Polymerpartikeln herausdiffundierende Monomerflüssigkeit wirkt bis zu ihrem Verbrauch infolge der Nachpolymerisation als Weichmacher. Dies führt dazu, dass die initial ausgehärteten Knochenzementpasten zwar die Anforderungen der ISO5833 erfüllen, jedoch noch eine ausgeprägte Nachhärtung infolge der Nachpolymerisation der aus den unlöslichen Polymerpartikeln diffundierenden Monomerflüssigkeit zeigen.

Die erfindungsgemäße Aufgabe besteht daher darin, einen Kit zur Herstellung von Knochenzement bereitzustellen, der eine hohe initiale Festigkeit aufweist und nur eine geringe Nachhärtung zeigt.

Ferner hat sich die Erfindung zur Aufgabe gestellt, ein Verfahren zur Herstellung eines solchen Knochenzements zur Verfügung zu stellen.

Diese Aufgaben werden gelöst durch die Gegenstände der unabhängigen Ansprüche.

Die Erfindung stellt daher einen Kit zur Herstellung von Knochenzement bereit, wenigstens aufweisend eine Paste A und eine Paste B, wobei
(a) Paste A
   (a1) wenigstens ein radikalisch polymerisierbares Monomer,
   (a2) wenigstens ein in (a1) unlösliches Polymer,
   (a3) wenigstens ein in (a1) lösliches Polymer und
   (a4) wenigstens einen radikalischen Polymerisationsinitiator enthält,
   wobei das Gewichtsverhältnis von dem wenigstens einen in (a1) unlöslichen Polymer (a2) zu dem wenigstens einen in (a1) löslichen Polymer (a3) wenigstens 2 zu 1 beträgt, und
(b) Paste B
   (b1) wenigstens ein radikalisch polymerisierbares Monomer,
   (b2) wenigstens ein in (b1) lösliches Polymer und
   (b3) wenigstens einen in (b1) löslichen Akzelerator enthält,
   (b4) gegebenenfalls ein in (b1) unlösliches Polymer,
   wobei der maximale Gehalt an in (b1) unlöslichem Polymer (b4) bei 5 Gewichtsprozent, bezogen auf das Gesamtgewicht von Paste B liegt, und
   wobei das Gewichtsverhältnis von in (b1) unlöslichem Polymer (b4) zu dem wenigstens einen in (b1) löslichen Polymer (b2) höchstens 0,2 beträgt.

Zudem stellt die Erfindung ein Verfahren zum Herstellen von Knochenzement bereit, bei dem
(i) ein Kit zur Herstellung von Knochenzement wenigstens aufweisend eine Paste A und eine Paste B, wobei
   (a) Paste A
      (a1) wenigstens ein radikalisch polymerisierbares Monomer,
      (a2) wenigstens ein in (a1) unlösliches Polymer,
      (a3) wenigstens ein in (a1) lösliches Polymer und
      (a4) wenigstens einen radikalischen Polymerisationsinitiator enthält,
      wobei das Gewichtsverhältnis von dem wenigstens einen in (a1) unlöslichen Polymer (a2) zu dem wenigstens einen in (a1) löslichen Polymer (a3) wenigstens 2 zu 1 beträgt, und
   (b) Paste B
      (b1) wenigstens ein radikalisch polymerisierbares Monomer,
      (b2) wenigstens ein in (b1) lösliches Polymer und
      (b3) wenigstens einen in (b1) löslichen Akzelerator enthält,
      (b4) gegebenenfalls ein in (b1) unlösliches Polymer,
      wobei der maximale Gehalt an in (b1) unlöslichem Polymer (b4) bei 5 Gewichtsprozent, bezogen auf das Gesamtgewicht von Paste B liegt, und
      wobei das Gewichtsverhättnis von in (b1) unlöslichem Polymer (b4) zu dem wenigstens einen in (b1) löslichen Polymer (b2) höchstens 0,2 beträgt, bereitgestelit wird und
(ii) die Pasten A und B miteinander vermischt werden.

Der erfindungsgemäße Kit zur Herstellung von Knochenzement weist demnach hinsichtlich der qualitativen und quantitativen Zusammensetzung einen asymmetrischen Aufbau auf. Eine erste Paste umfasst ein radikalisch polymerisierbares Monomer, ein in diesem Monomer unlösliches Polymer und ein in diesem Monomer lösliches Polymer. In dieser ersten Paste ist zudem ein Polymerisationsinitiator enthalten. Die erste Paste weist einen hohen Anteil an in dem Monomer unlöslichem Polymer auf. Eine zweite Paste umfasst ein radikalisch polymerisierbares Monomer, ein in diesem Monomer lösliches Polymer und einen Akzelerator. Die zweite Paste weist kein im Monomer unlösliches Polymer oder nur einen geringen Anteil an im Monomer unlöslichem Polymer auf.

Dieser asymmetrische Aufbau hat eine besondere technische Wirkung zur Folge: Durch die Gegenwart des Polymerisationsinitiators in der ersten Paste, die hohe Mengen an unlöslichem Polymer enthält, kann das unlösliche Polymer das radikalisch polymerisierbare Monomer mit dem darin gelösten Polymerisationsinitiator einschließen. Ein Einschluss des Akzelerators in unlösliches Polymer ist dagegen praktisch nicht möglich, da der Akzelerator nur in der zweiten Paste enthalten ist und in dieser das unlösliche Polymer überhaupt nicht oder nur in geringen Mengen vorhanden ist. Nach dem Vermischen der beiden Pasten beginnt daher die Polymerisation innerhalb einer Phase, die das radikalisch polymerisierbare Monomer, das darin gelöstes Polymer, den Polymerisationsinitiator und den Akzelerator aufweist. Da der Akzelerator nur in der zweiten Paste vorgesehen ist, die vollständig oder weitestgehend frei von unlöslichem Polymer ist, ist die gesamte Menge an Akzelerator in der Mischung aus beiden Pasten homogen verteilt. Eine Nachdiffusion von radikalisch polymerisierbarem Monomer und dem Akzelerator ist praktisch ausgeschlossen, da kein oder nahezu kein unlösliches Polymer zum Einschließen des radikalisch polymerisierbaren Monomers und des Akzelerators bereitsteht. Dies hat zur Folge, dass aus dem in der Mischung aus beiden Pasten enthaltenen unlöslichen Polymer nur noch radikalisch polymerisierbares Monomer mit darin gelöstem Polymerisationsinitiator austreten kann. In diesem Fall wird das radikalisch polymerisierbare Monomer unmittelbar unter Mitwirkung des in der umgebenden Matrix vorhandenen Akzelerators polymerisiert. Darüber hinaus kann der Akzelerator in die Partikel aus unlöslichem Polymer diffundieren und die darin eingeschlossenen Reste an radikalisch polymerisierbarem Monomer in Gegenwart des ebenfalls anwesenden Polymerisationsinitiators polymerisieren. Durch die vorliegende Erfindung wird daher in praktisch erheblichem Maße ausgeschlossen, dass noch nicht polymerisiertes Monomer aus den Partikeln des unlöslichen Polymers austreten kann. Damit steht das radikalisch polymerisierbare Monomer nicht als Weichmacher zur Verfügung, wodurch die Nachhärtung des Knochenzements deutlich verringert werden kann.

Die vorliegende Erfindung stellt einen Kit zur Herstellung von Knochenzement bereit.

Erfindungsgemäß wird unter Kit ein System aus wenigstens zwei Komponenten verstanden. Obwohl im Folgenden von zwei Komponenten die Rede ist, kann der Kit gegebenenfalls auch mehr als zwei Komponenten, beispielsweise drei, vier, fünf oder mehr als fünf Komponenten enthalten. Die einzelnen Kitkomponenten liegen vorzugsweise getrennt voneinander verpackt vor, so dass die Inhaltsstoffe der einen Kitkomponente mit den Inhaltsstoffen einer weiteren Kitkomponente nicht in Kontakt stehen. Beispielsweise ist es möglich, die jeweiligen Kitkomponenten getrennt voneinander zu verpacken und zusammen in einem Vorratsbehälter aufzubewahren.

Gemäß einer bevorzugten Ausführungsform wird der Kit durch eine Vorrichtung zur Herstellung von Knochenzement verwirklicht. Eine solche Vorrichtung zur Herstellung von Knochenzement kann beispielsweise wenigstens zwei Behälter umfassen, wobei in einem ersten Behälter die Paste A und in einem zweiten Behälter die Paste B enthalten ist. Wenigstens einer der beiden Behälter ist vorzugsweise von einem Anwender öffenbar, um nach dem Öffnen ein Vermischen der Pasten A und B zu gestatten. Ferner ist die Vorrichtung zur Herstellung von Knochenzement vorzugsweise so ausgestaltet, dass nach dem Öffnen wenigstens eines der beiden Behälter die in den beiden Behältern enthaltenen Pasten miteinander in Kontakt treten können. Neben den beiden Behältern kann die Vorrichtung zur Herstellung von Knochenzement insbesondere noch eine Mischeinheit zum Vermischen der Pasten A und B zu einem Mischgut enthalten. Beispielsweise kann die Vorrichtung zur Herstellung von Knochenzement durch eine Doppelkartusche verwirklicht sein, in der die Pasten A und B getrennt voneinander bereitgestellt werden. Auf dieser Doppelkartusche kann gegebenenfalls eine Mischeinheit wie ein statischer oder aktiv angetriebener Mischer angeordnet sein, um eine Vermischung der Pasten A und B zu erreichen.

Der Kit weist erfindungsgemäß wenigstens eine Paste A und eine Paste B auf.

Dieses wenigstens eine radikalisch polymerisierbare Monomer (a1) weist vorzugsweise einen pH-Wert in Wasser im Bereich von 5-9 auf. Das radikalisch polymerisierbare Monomer (a1) ist bei einer Temperatur von 25°C und einem Druck von 1013 hPa vorzugsweise flüssig. Gemäß einer bevorzugten Ausführungsform ist das radikalisch polymerisierbare Monomer (a1) destillierbar.

Das radikalisch polymerisierbare Monomer (a1) ist vorzugsweise ein Methacrylsäureester. Vorzugsweise handelt es sich bei dem Methacrylsäureester um einen monofunktionellen, difunktionellen oder trifunktionellen Methacrylsäureester.

Bei dem Methacrylsäureester handelt es sich vorzugsweise um einen aliphatischen Methacrylsäureester und mehr bevorzugt um einen Methacrylsäurealkylester. Diese Methacrylsäurealkylester sind gemäß einer bevorzugten Ausführungsform Ester von Methacrylsäure mit Alkoholen, die 1-20 Kohlenstoffatome, mehr bevorzugt 1-10 Kohlenstoffatome, noch mehr bevorzugt 1 - 6 Kohlenstoffatome und ganz besonders bevorzugt 1 - 4 Kohlenstoffatome aufweisen. Die Alkohole können substituiert oder nicht substituiert sein und sind vorzugsweise nicht substituiert. Die Alkohole können ferner gesättigt oder ungesättigt sein und sind vorzugsweise gesättigt. Bei den Alkoholen kann es sich um Monoalkohole, Dialkohole oder Polyalkohole handeln.

Gemäß einer besonders bevorzugten Ausführungsform ist das radikalisch polymerisierbare Monomer (a1) aus der Gruppe ausgewählt, die aus Methacrylsäuremethylester, Methacrylsäureethylester, Ethylenglykoldimethacrylat und Butan-1,4-dioldimethacrylat besteht.

Das erfindungsgemäß eingesetzte radikalisch polymerisierbare Monomer (a1) weist vorzugsweise eine Molmasse von weniger als 1000 g/mol auf. Davon umfasst sind auch radikalisch polymerisierbare Monomere, die Bestandteil einer Monomermischung sind, wobei wenigstens Das erfindungsgemäß eingesetzte radikalisch polymerisierbare Monomer (a1) weist vorzugsweise eine Molmasse von weniger als 1000 g/mol auf. Davon umfasst sind auch radikalisch polymerisierbare Monomere, die Bestandteil einer Monomermischung sind, wobei wenigstens eines der radikalisch polymerisierbaren Monomere der Monomermischung eine definierte Struktur mit einer Molmasse von weniger als 1000 g/mol aufweist.

Paste A enthält vorzugsweise 15-75 Gewichtsprozent, mehr bevorzugt 15-70 Gewichtsprozent, noch mehr bevorzugt 20 - 60 Gewichtsprozent und besonders bevorzugt 25 - 50 Gewichtsprozent wenigstens eines radikalisch polymerisierbaren Monomers (a1), bezogen auf das Gesamtgewicht von Paste A.

Paste A enthält ferner wenigstens ein in (a1) unlöslichen Polymer (a2).

Das in (a1) unlösliche Polymer (a2) ist vorzugsweise partikulär. Gemäß einer besonders bevorzugten Ausführungsform weist das in (a1) unlösliche Polymer (a2) eine durchschnittliche Teilchengröße im Bereich von 10 nm - 500 µm und besonders bevorzugt im Bereich von 100 nm - 500 µm auf. Unter durchschnittlicher Teilchengröße wird vorliegend ein Größenbereich verstanden, der von wenigstens 90 Prozent der Teilchen eingenommen wird.

Das in (a1) unlösliche Polymer (a2) weist vorzugsweise eine gewichtsmittlere Molmasse von wenigstens 150.000 g/mol und mehr bevorzugt eine gewichtsmittlere Molmasse von wenigstens 500.000 g/mol auf. Die Angabe der Molmasse bezieht sich auf die viskosimetrisch bestimmte Molmasse.

Das in (a1) unlösliche Polymer (a2) kann vernetzt oder unvernetzt sein und ist vorzugsweise vernetzt. Die Vernetzung erfolgt dabei vorzugsweise mit einer difunktionellen Verbindung. Die difunktionelle Verbindung kann beispielsweise aus der Gruppe ausgewählt sein, die aus Alkylenglykoldimethacrylaten besteht. Als Vernetzter hat sich beispielsweise Ethylenglykoldimethacrylat bewährt

Bei dem In (a1) unlöslichen Polymer (a2) kann es sich um ein Homopolymer oder um ein Copolymer handeln.

Vorzugsweise handelt es sich bei dem in (a1) unlöslichen Polymer (a2) um ein Polymer eines Methacrylsäureesters. Gemäß einer bevorzugten Ausführungsform ist das in (a1) unlösliche Polymer (a2) ein Homopolymer oder ein Copolymer eines Methacrylsäurealkylesters.

Gemäß einer besonders bevorzugten Ausführungsform ist das wenigstens eine in (a1) unlösliche Polymer (a2) aus der Gruppe ausgewählt, die aus vernetztem Poly(methylmethacrylat-co-methylacrylat) und vernetztem Poly(methylmethacrylat) besteht.

Das Polymer (a2) ist in dem wenigstens einen radikalisch polymerisierbaren Monomer (a1) unlöslich. Erfindungsgemäß ist das Polymer (a2) in dem wenigstens einen radikalisch polymerisierbaren Monomer (a1) unlöslich, wenn das Polymer (a2) bei einer Temperatur von 25°C eine Löslichkeit im radikalisch polymerisierbaren Monomer (a1) von weniger als 50 g/l, vorzugsweise von weniger als 25 g/l, mehr bevorzugt von weniger als 10 g/l und noch mehr bevorzugt von weniger als 5 g/l aufweist.

Der Anteil des wenigstens einen in (a1) unlöslichen Polymers (a2) liegt vorzugsweise im Bereich von 20 - 70 Gewichtsprozent, mehr bevorzugt im Bereich von 25 - 60 Gewichtsprozent, noch mehr bevorzugt im Bereich von 30 - 55 Gewichtsprozent und besonders bevorzugt im Bereich von 34-47 Gewichtsprozent, bezogen auf das Gesamtgewicht von Paste A.

Die Paste A enthält außerdem wenigstens ein in (a1) lösliches Polymer (a3).

Bei dem in (a1) löslichen Polymer (a3) handelt es sich vorzugsweise um ein Polymer mit einer gewichtsmittleren Molmasse von weniger als 500.000 g/mol und mehr bevorzugt um ein Polymer mit einer gewichtsmittleren Molmasse von weniger als 150.000 g/mol. Die Angabe der Molmasse bezieht sich auf die viskosimetrisch bestimmte Molmasse.

Das in (a1) unlösliche Polymer (a3) kann vernetzt oder unvernetzt sein und ist vorzugsweise unvernetzt.

Das in (a1) lösliche Polymer (a3) kann ein Homopolymer oder ein Copolymer sein.

Vorzugsweise ist das wenigstens eine im radikalisch polymerisierbaren Monomer (a1) lösliche Polymer (a3) ein Polymer eines Methacrylsäureesters. Gemäß einer besonders bevorzugten Ausführungsform ist das wenigstens eine im radikalisch polymerisierbaren Monomer (a1) lösliche Polymer (a3) ein Copolymer von Methacrylsäuremethylester.

Gemäß einer weiteren besonders bevorzugten Ausführungsform ist das wenigstens eine in (a1) lösliche Polymer (a3) aus der Gruppe ausgewählt, die aus Poly(methacrylsäuremethylester) (PMMA), Poly(methacrylsäureethylester) (PMAE), Poly(methacrylsäurepropylester) (PMAP), Poly(methacrylsäureisopropylester), Poly(methylmethacrylat-co-methylacrylat) und Poly(styrol-co-methylmethacrylat) besteht.

Das Polymer (a3) ist in dem wenigstens einen radikalisch polymerisierbaren Monomer (a1) löslich. Erfindungsgemäß ist das Polymer (a3) in dem wenigstens einen radikalisch polymerisierbaren Monomer (a1) löslich, wenn das Polymer (a3) Im radikalisch polymerisierbaren Monomer (a1) bei einer Temperatur von 25°C eine Löslichkeit von wenigstens 25 g/l, mehr bevorzugt eine Löslichkeit von wenigstens 50 g/l und ganz besonders bevorzugt eine Löslichkeit von wenigtens 100 g/l aufweist.

Der Anteil des in (a1) löslichen Polymers (a3) liegt vorzugsweise im Bereich von 1-25 Gewichtsprozent, mehr bevorzugt im Bereich von 2-20 Gewichtsprozent, noch mehr bevorzugt im Bereich von 2-18 Gewichtsprozent und besonders bevorzugt im Bereich von 3-16 Gewichtsprozent, bezogen auf das Gesamtgewicht von Paste A.

Das Gewichtsverhältnis von dem wenigstens einen in (a1) unlöslichen Polymer (a2) zu dem wenigstens einen in (a1) löslichen Polymer (a3) beträgt in Paste A wenigstens 2 zu 1. Gemäß einer bevorzugten Ausführungsform beträgt in Paste A das Gewichtsverhältnis von dem wenigstens einen in (a1) unlöslichen Polymer (a2) zu dem wenigstens einen in (a1) löslichen Polymer (a3) wenigstens 2,1 zu 1,0, mehr bevorzugt wenigstens 2,2 zu 1,0 und noch mehr bevorzugt wenigstens 2,3 zu 1,0.

Paste A enthält einen radikalischen Polymerisationsinitiator (a4).

Der radikalische Polymerisationsinitiator (a4) ist vorzugsweise in dem wenigstens einen radikalisch polymerisierbaren Monomer (a1) löslich. Erfindungsgemäß ist der radikalische Polymerisationsinitiator (a4) in dem wenigstens einen radikalisch polymerisierbaren Monomer (a1) löslich, wenn der radikalische Polymerisationsinitiator (a4) im radikalisch polymerisierbaren Monomer (a1) bei einer Temperatur von 25°C eine Löslichkeit von wenigstens 25 g/l, mehr bevorzugt eine Löslichkeit von wenigstens 50 g/l und ganz besonders bevorzugt eine Löslichkeit von wenigstens 100 g/l aufweist.

Es kann vorteilhaft sein, wenn als radikalischer Polymerisationsinitiator (a4) eine Verbindung verwendet wird, die sowohl akzeleratorinduziert als auch bei thermischer Belastung unter Radikalbildung zerfallen kann.

Gemäß einer bevorzugten Ausführungsform handelt es sich bei dem radikalischen Polymerisationsinitiator (a4) um ein Peroxid. Unter den Begriff Peroxide fallen Verbindungen, die wenigstens eine Peroxygruppe -O-O- enthalten. Für die Polymerisation von radikalisch polymerisierbaren Monomeren geeignete Polymerisationsinitiatoren sind dem Fachmann bekannt. Als geeignete Peroxide haben sich beispielsweise Dibenzoylperoxid und Cumolhydroperoxid erwiesen.

Gemäß einer weiteren bevorzugten Ausführungsform handelt es sich bei dem radikalischen Polymerisationsinitiator (a4) um ein Barbitursäurederivat. Das Barbitursäurederivat kann beispielsweise aus der Gruppe ausgewählt sein, die aus 1,5-disubstituierten Barbituraten, 1,3,5-trisubtituierten Barbituraten und 1,3,5-tetrasubtituierten Barbituraten besteht. Die Art der Substituenten an der Barbitursäure ist dabei nicht weiter eingeschränkt. Bei den Substituenten kann es sich beispielsweise um aliphatische oder aromatische Substituenten handeln. Hierbei können alkylische, cycloalkylische, allylische oder arylische Substituenten bevorzugt sein. Die Substituenten können auch Heteroatome tragen. Insbesondere kann es sich bei den Substituenten um Thiosubstituenten handeln. Demnach können 1,5-disubstituierte Thiobarbiturate oder 1,3,5-trisubstituierte Thiobarbiturate bevorzugt sein. Bevorzugt verwendbar sind Barbiturate, die an Position 1 und an Position 5 jeweils einen Substituenten, an den Positionen 1, 3 und 5 jeweils einen Substituenten oder an den Positionen 1 und 3 jeweils einen Substituenten und an Position 5 zwei Substituenten tragen. Gemäß einer bevorzugten Ausführungsform ist das Barbitursäure-Derivat ein 1,5-disubstituiertes Barbiturat oder ein 1,3,5-trisubtituiertes Barbiturat, so zum Beispiel ein 1,5-Dialkylbarbiturat, ein 1-Cycloalkyl-5-alkylbarbiturat oder ein 1-Aryl-5-alkylbarbiturat. 1,3,5-tetrasubtituierte Barbiturate können ebenfalls verwendet werden, weisen jedoch die Eigenschaft auf, die Blut-Hirn-Schranke überwinden zu können und daher pharmakologisch aktiv zu sein. Gemäß einer besonders bevorzugten Ausführungsform ist das Barbitursäurederivat aus der Gruppe ausgewählt, die aus 1-Cyclohexyl-5-ethylbarbitursäure, 1-Phenyl-5-ethylbarbitursäure, 1-Benzyl-5-ethylbarbftursäute und 1,3,5-Trimethylbarbitursäure besteht. bevorzugt im Bereich von 0,1-10 Gewichtsprozent, bezogen auf das Gesamtgewicht von Paste A.

Paste B enthält wenigstens ein radikalisch polymerisierbares Monomer (b1).

Dieses wenigstens eine radikalisch polymerisierbare Monomer (b1) weist vorzugsweise einen pH-Wert in Wasser im Bereich von 5-9 auf. Das radikalisch polymerisierbare Monomer (b1) ist bei einer Temperatur von 25°C und einem Druck von 1013 hPa vorzugsweise flüssig. Gemäß einer bevorzugten Ausführungsform ist das radikalisch polymerisierbare Monomer (b1) destillierbar.

Das radikalisch polymerisierbare Monomer (b1) ist vorzugsweise ein Methacrylsäureester. Vorzugsweise handelt es sich bei dem Methacrylsäureester um einen monofunktionellen, difunktionellen oder trifunktionellen Methacrylsäureester.

Bei dem Methacrylsäureester handelt es sich vorzugsweise um einen aliphatischen Methacrylsäureester und mehr bevorzugt um einen Methacrylsäurealkylester. Diese Methacrylsäurealkylester sind gemäß einer bevorzugten Ausführungsform Ester von Methacrylsäure mit Alkoholen, die 1 - 20 Kohlenstoffatome, mehr bevorzugt 1-10 Kohlenstoffatome, noch mehr bevorzugt 1-6 Kohlenstoffatome und ganz besonders bevorzugt 1 - 4 Kohlenstoffatome aufweisen. Die Alkohole können substituiert oder nicht substituiert sein und sind vorzugsweise nicht substituiert. Die Alkohole können ferner gesättigt oder ungesättigt sein und sind vorzugsweise gesättigt. Bei den Alkoholen kann es sich um Monoalkohole, Dialkohole oder Polyalkohole handeln.

Gemäß einer besonders bevorzugten Ausführungsform ist das radikalisch polymerisierbare Monomer (b1) aus der Gruppe ausgewählt, die aus Methacrylsäuremethylester, Methacrylsäureethylester, Ethylenglykoldimethacrylat und Butan-1,4-dioldimethacrylat besteht

Das erfindungsgemäß eingesetzte radikalisch polymerisierbare Monomer (b1) weist vorzugsweise eine Molmasse von weniger als 1000 g/mol auf. Davon umfasst sind auch radikalisch polymerisierbare Monomere, die Bestandteil einer Monomermischung sind, wobei wenigstens eines der radikalisch polymerisierbaren Monomere der Monomermischung eine definierte Struktur mit einer Molmasse von weniger als 1000 g/mol aufweist.

Paste B enthält vorzugsweise 10-70 Gewichtsprozent, mehr bevorzugt 15-60 Gewichtsprozent, noch mehr bevorzugt 20 - 55 Gewichtsprozent und besonders bevorzugt 25 - 50 Gewichtsprozent wenigstens eines radikalisch polymerisierbaren Monomers (b1), bezogen auf das Gesamtgewicht von Paste B.

Die Paste B enthält außerdem wenigstens ein in (b1) lösliches Polymer (b2).

Bei dem in (b1) löslichen Polymer (b2) handelt es sich vorzugsweise um ein Polymer mit einer gewichtsmittleren Molmasse von weniger als 500.000 g/mol und mehr bevorzugt um ein Polymer mit einer gewichtsmittieren Molmasse von weniger als 150.000 g/mol. Die Angabe der Molmasse bezieht sich auf die viskosimetrisch bestimmte Molmasse.

Das in (b1) unlösliche Polymer (b2) kann vernetzt oder unvernetzt sein und ist vorzugsweise unvernetzt.

Das in (b1) lösliche Polymer (b2) kann ein Homopolymer oder ein Copolymer sein.

Vorzugsweise ist das wenigstens eine im radikalisch polymerisierbaren Monomer (b1) lösliche Polymer (b2) ein Polymer eines Methacrylsäureesters. Gemäß einer besonders bevorzugten Ausführungsform ist das wenigstens eine im radikalisch polymerisierbaren Monomer (b1) lösliche Polymer (b2) ein Copolymer von Methacrylsäuremethylester.

Gemäß einer weiteren besonders bevorzugten Ausführungsform ist das wenigstens eine in (b1) lösliche Polymer (b2) aus der Gruppe ausgewählt, die aus Poly(methacrylsäuremethylester) (PMMA), Poly(methacrylsäureethylester) (PMAE), Poly(methacrylsäurepropylester) (PMAP), Poly(methacrylsäureisopropylester), Poly(methylmethacrylat-co-methylacrylat) und Poly(styrol-co-methylmethacrylat) besteht.

Das Polymer (b2) ist in dem wenigstens einen radikalisch polymerisierbaren Monomer (b1) löslich. Erfindungsgemäß ist das Polymer (b2) in wenigstens einen radikalisch polymerisierbaren Monomer (b1) löslich, wenn das Polymer (b2) im radikalisch polymerisierbaren Monomer (b1) bei einer Temperatur von 25°C eine Löslichkeit von wenigstens 25 g/l, mehr bevorzugt eine Löslichkeit von wenigstens 50 g/l und ganz besonders bevorzugt eine Löslichkeit von wenigstens 100 g/l aufweist.

Der Anteil des in (b1) löslichen Polymers (b2) liegt vorzugsweise im Bereich von 25 - 85 Gewichtsprozent, mehr bevorzugt im Bereich von 35 - 85 Gewichtsprozent, noch mehr bevorzugt im Bereich von 40 - 80 Gewichtsprozent und besonders bevorzugt im Bereich von 50 - 75 Gewichtsprozent, bezogen auf das Gesamtgewicht von Paste B.

Paste B enthält zudem wenigstens einen Akzelerator (b3).

Bei dem Akzelerator (b3) kann es sich um einen der fachüblichen Akzeleratoren handeln.

Gemäß einer Ausführungsform ist der Akzelerator (b3) aus der Gruppe ausgewählt, die aus N,N-Dimethyl-p-toluidin, N,N-Bis-hydroxyethyl-p-toluidin, N,N-Dimethyl-anilin, 4-N,N-Dimethylamino-pyridin, Saccharin, Lithiumchlorid, Trioctylmethylammoniumchlorid und Mischungen davon besteht. Ein solcher Akzelerator (b3) wird vorzugsweise dann verwendet, wenn in Paste A des Kits als Polymerisationsinitiator (a4) ein Peroxid eingesetzt wird.

Gemäß einer weiteren Ausführungsform handelt es sich bei dem Akzelerator (b3) um ein organisches (II)-Kupfersalz. Vorzugsweise ist der Akzelerator (b3) in diesem Fall aus der Gruppe ausgewählt, die aus Kupfer(II)-2-ethylhexanoat, Kupfer(II)-methacrylat, Kupfer(II)-acetylacetonat, basischem Kupfer(II)-carbonat und Kupfer(11)-hydroxid besteht. Ein solcher Akzelerator (b3) wird vorzugsweise dann verwendet, wenn in Paste A des Kits als Polymerisationsinitiator (a4) ein Barbitursäurederivat eingesetzt wird. In diesem Fall kann es ferner von Vorteil sein, dass wenigstens eine der Pasten A und B, vorzugsweise Paste B, ein Halogenidsalz enthält. Bei diesem Halogenidsalz kann es sich beispielsweise um ein anorganisches oder ein organisches Salz von Chlor oder Brom handeln. Die Verwendung von quartären Alkyl-, Aryl-, Aryldialkyl-, Diarylalkyl- oder Cycloalkyldialkylammoniumsalzen, wie zum Beispiel Trioctylmethylammoniumchlorid, hat sich als besonders vorteilhaft erwiesen. Ebenso können jedoch auch Hydrohalogenide oder Metallhalogenide verwendet werden.

Der Anteil des wenigstens einen Akzelerators (b3) liegt vorzugsweise im Bereich von 0,00001 - 15 Gewichtsprozent, mehr bevorzugt im Bereich von 0,001 -10 Gewichtsprozent, noch mehr bevorzugt im Bereich von 0,01 -10 Gewichtsprozent und besonders bevorzugt im Bereich von 0,1-10 Gewichtsprozent, bezogen auf das Gesamtgewicht von Paste B.

Gegebenenfalls ist in Paste B ein in (b1) unlösliches Polymer (b4) enthalten. In Paste B beträgt der maximale Gehalt an in (b1) unlöslichem Polymer (b4) 5 Gewichtsprozent, bezogen auf das Gesamtgewicht von Paste B. Demnach kann in Paste B erfindungsgemäß kein in (b1) unlösliches Polymer (b4) enthalten sein. Andererseits ist es ebenso möglich, dass in Paste B geringe Mengen an in (b1) unlöslichem Polymer (b4) enthalten sind, solange der Gehalt an in (b1) unlöslichem Polymer (b4) 5 Gewichtsprozent, bezogen auf das Gesamtgewicht von Paste B, nicht übersteigt Gemäß einer bevorzugten Ausführungsform beträgt der maximale Gehalt an in (b1) unlöslichem Polymer (b4) 5 Gewichtsprozent, mehr bevorzugt 4 Gewichtsprozent, noch mehr bevorzugt 3 Gewichtsprozent, besonders bevorzugt 2 Gewichtsprozent und ganz besonders bevorzugt 1 Gewichtsprozent, bezogen auf das Gesamtgewicht von Paste B. Gemäß einer ganz besonders bevorzugten Ausführungsform beträgt der Gehalt an in (b1) unlöslichem Polymer (b4) jedoch 0 Gewichtsprozent, bezogen auf das Gesamtgewicht von Paste B.

In Paste B beträgt das Gewichtsverhältnis von in (b1) unlöslichem Polymer (b4) zu dem wenigstens einen in (b1) löslichen Polymer (b2) höchstens 0,2. Vorzugsweise beträgt das Gewichtsverhältnis von in (b1) unlöslichem Polymer (b4) zu dem wenigstens einen in (b1) löslichen Polymer (b2) höchstens 0,15, mehr bevorzugt höchstens 0,1, noch mehr bevorzugt höchstens 0,05, besonders bevorzugt höchstens 0,02 und ganz besonders bevorzugt 0.

Das in (b1) unlösliche Polymer (b4) kann partikulär sein. Es kann eine durchschnittliche Teilchengröße im Bereich von 10 nm - 500 oder im Bereich von 100 nm -500 µm aufweisen. Unter durchschnittlicher Teilchengröße wird vorliegend ein Größenbereich verstanden, der von wenigstens 90 Prozent der Teilchen eingenommen wird.

Das in (b1) unlösliche Polymer (b4) kann eine gewichtsmittlere Molmasse von wenigstens 150.000 g/mol oder von wenigstens 500.000 g/mol aufweisen. Die Angabe der Molmasse bezieht sich auf die viskosimetrisch bestimmte Molmasse.

Das in (b1) unlösliche Polymer (b4) kann vernetzt oder unvernetzt sein. Die Vernetzung kann dabei beispielsweise mit einer difunktionellen Verbindung erfolgen. Die difunktionelle Verbindung kann zum Beispiel aus der Gruppe ausgewählt sein, die aus Alkylenglykoldimethacrylaten besteht. Als Vernetzter kommt daher beispielsweise Ethylenglykoldimethacrylat in Betracht.

Bei dem in (b1) unlöslichen Polymer (b4) kann es sich um ein Homopolymer oder um ein Copolymer handeln.

Das in (b1) unlösliche Polymer (b4) kann ein Polymer eines Methacrylsäureesters sein. Beispielsweise kann das in (b1) unlösliche Polymer (b4) ein Homopolymer oder ein Copolymer eines Methacrylsäurealkylesters sein.

Das wenigstens eine in (b1) unlösliche Polymer (b4) kann aus der Gruppe ausgewählt sein, die aus vernetztem Poly(methylmethacrylat-co-methylacrylat) und vernetztem Poly(methylmethacrylat) besteht.

Das Polymer (b4) ist in dem wenigstens einen radikalisch polymerisierbaren Monomer (b1) unlöslich. Erfindungsgemäß ist das Polymer (b4) in dem wenigstens einen radikalisch polymerisierbaren Monomer (b1) unlöslich, wenn das Polymer (b4) bei einer Temperatur von 25°C eine Löslichkeit im radikalisch polymerisierbaren Monomer (b1) von weniger als 50 g/l, vorzugsweise von weniger als 25 g/l, mehr bevorzugt von weniger als 10 g/l und noch mehr bevorzugt von weniger als 5 g/l aufweist.

Neben den vorstehend erläuterten Bestandteilen können die Pasten A und B weitere Bestandteile aufweisen. Diese weiteren Bestandteile können jeweils entweder in Paste A, in Paste B oder in den Pasten A und B enthalten sein.

Gemäß einer bevorzugten Ausführungsform ist in wenigstens einer der Pasten A und B wenigstens ein Röntgenopaker enthalten. Beim Röntgenopaker kann es sich um einen fachüblichen Röntgenopaker handeln. Geeignete Röntgenopaker können in dem radikalisch polymerisierbaren Monomer (a1) oder dem radikalisch polymerisierbaren Monomer (b1) löslich oder unlöslich sein. Der Röntgenopaker ist vorzugsweise aus der Gruppe ausgewählt, die aus Metalloxiden (wie zum Beispiel Zirkoniumdioxid), Bariumsulfat, toxikologisch unbedenklichen Schwermetallpartikeln (wie zum Beispiel Tantal), Ferrit, Magnetit (ggf. auch supramagnetisches Magnetit) und biokompatiblen Calciumsalzen besteht Diese Röntgenopaker weisen vorzugsweise einen mittleren Teilchendurchmesser im Bereich von 10 nm - 500 µm auf. Als Röntgenopaker kommen ferner auch Ester der 3,5-Bis(acetamido)-2,4,6-triodbenzoesäure, Gadolinium-Verbindungen, wie Gadolinium-Chelat mit den Estern der 1,4,7,10-Tetraazacyclododecan-1,4,7,10-tetraessigsäure (DOTA), in Betracht.

Gemäß einer weiteren bevorzugten Ausführungsform ist in wenigstens einer der Pasten A und B wenigstens ein Farbstoff enthalten. Der Farbstoff kann ein fachüblicher Farbstoff und vorzugsweise ein Lebensmittelfarbstoff sein. Ferner kann der Farbstoff in dem wenigstens einen radikalisch polymerisierbaren Monomer (a1) oder in dem wenigstens einen radikalisch polymerisierbaren Monomer (a2) löslich oder unlöslich sein. Gemäß einer besonders bevorzugten Ausführungsform wird der Farbstoff aus der Gruppe ausgewählt, die aus E101, E104, E132, E141 (Chlorophyllin), E142, Riboflavin und Lissamingrün besteht. Unter den Begriff Farbstoff fallen erfindungsgemäß auch Farblacke, wie zum Beispiel Farblack Grün, das Aluminiumsalz einer Mischung aus E104 und E132.

Gemäß einer weiteren bevorzugten Ausführungsform ist in wenigstens einer der Pasten A und B wenigstens ein pharmazeutischer Wirkstoff enthalten.

Der wenigstens eine pharmazeutische Wirkstoff kann in wenigstens einer der Pasten A und B in gelöster oder suspendierter Form enthalten sein.

Der pharmazeutische Wirkstoff kann vorzugsweise aus der Gruppe ausgewählt sein, die aus Antibiotika, Aniphlogistika, Steroiden, Hormonen, Wachstumsfaktoren, Bisphosphonaten, Cytostatika und Genvektoren besteht. Gemäß einer besonders bevorzugten Ausführungsform handelt es sich bei dem wenigstens einen pharmazeutischen Wirkstoff um ein Antibiotikum.

Das wenigstens eine Antibiotikum ist vorzugsweise aus der Gruppe ausgewählt, die aus Aminoglykosid-Antibiotika, Glykopeptid-Antibiotika, Lincosamid-Antibiotika, Gyrasehemmern, Carbapenemen, cyclischen Lipopeptiden, Glycylcyclinen, Oxazolidonen und Polypeptidantibiotika besteht.

Gemäß einer besonders bevorzugten Ausführungsform handelt es sich bei dem wenigstens einen Antibiotikum um ein Mitglied, das aus der Gruppe ausgewählt ist, die aus Gentamicin, Tobramycin, Amikacin, Vancomycin, Teicoplanin, Dalbavancin, Lincosamin, Clindamycin, Moxifloxacin, Levofloxacin, Ofloxacin, Ciprofloxacin, Doripenem, Meropenem, Tigecyclin, Linezolid, Eperezolid, Ramoplanin, Metronidazol, Tinidazol, Omidazol und Colistin sowie Salzen und Estern davon besteht.

Demnach kann das wenigstens eine Antibiotikum aus der Gruppe ausgewählt sein, die aus Gentamicinsulfat, Gentamicinhydrochtorid, Amikacinsulfat, Amikacinhydrochlorid, Tobramycinsulfat, Tobramycinhydrochlorid, Clindamycinhydrochiorid, Lincosaminhydrochlorid und Moxifloxacin besteht.

Das wenigstens eine Antiphlogistikum ist vorzugsweise aus der Gruppe ausgewählt, die aus nicht-steroidalen Antiphlogistika und Glukokortikoiden besteht. Gemäß einer besonders bevorzugten Ausführungsform ist das wenigstens eine Antiphlogistikum aus der Gruppe ausgewählt, die aus Acetylsalicylsäure, Ibuprofen, Diclofenac, Ketoprofen, Dexamethason, Prednison, Hydrocortison, Hydrocortisonacetat und Fluticason besteht.

Das wenigstens eine Hormon ist vorzugsweise aus der Gruppe ausgewählt, die aus Serotonin, Somatotropin, Testosteron und Östrogen besteht.

Der wenigstens eine Wachstumsfaktor ist vorzugsweise aus der Gruppe ausgewählt, die aus dem *Fibroblast Growth Factor* (FGF), dem *Transforming Growth Factor* (TGF), dem *Platelet Derived Growth Factor* (PDGF), dem Epidermalen Wachstumsfaktor (EGF), dem *Vascular Endotheliel Growth Factor* (VEGF), insulinähnlichen Wachstumsfaktoren (IGF), dem *Hepatocyte Growth Factor* (HGF), dem *Bone Morphogenetic Protein* (BMP), Interleukin-18, Interleukin 8 und dem Nervenwachstumsfaktor besteht.

Das wenigstens eine Cytostatikum ist vorzugsweise aus der Gruppe ausgewählt, die aus Alkylantien, Platinanaloga, lnterkalantien, Mitosehemmem, Taxanen, Topoisomerasehemmem und Antimetaboliten besteht.

Das wenigstens eine Bisphosphonat ist vorzugsweise aus der Gruppe ausgewählt, die aus Zoledronat und Aledronat besteht.

Gemäß einer weiteren bevorzugten Ausführungsform ist in wenigstens einer der Pasten A und B wenigstens ein biokompatibles Elastomer enthalten. Das biokompatible Elastomer ist vorzugsweise partikulär. Vorzugsweise ist das biokompatible Elastomer in dem wenigstens einen radikalisch polymerisierbaren Monomer (a1) oder in dem wenigstens einen radikalisch polymerisierbaren Monomer (b1) löslich. Als besonders geeignet hat sich die Verwendung von Polybutadien als biokompatibles Elastomer erwiesen.

Gemäß einer weiteren bevorzugten Ausführungsform enthält wenigstens eine der Pasten A und B wenigstens ein Monomer mit Haftgruppen. Bei der Haftgruppe kann es sich beispielsweise um eine Amidgruppe handeln. Das Monomer mit Haftgruppe kann daher zum Beispiel Methacrylsäureamid sein. Durch die Verwendung von wenigstens einem Monomer mit Haftgruppen kann die Anbindung des Knochenzementes an Gelenkendoprothesen gezielt beeinflusst werden.

Gemäß einer weiteren bevorzugten Ausführungsform ist in wenigstens einer der Pasten A und B wenigstens ein Stabilisator enthalten. Der Stabilisator soll geeignet sein, eine Spontanpolymerisation der in den Pasten A und B enthaltenen polymerisierbaren Monomere zu verhindern. Ferner soll der Stabilisator keine störenden Wechselwirkungen mit den anderen in den Pasten enthaltenen Bestandteilen zeigen. Derartige Stabilisatoren sind aus dem Stand der Technik bekannt. Gemäß einer bevorzugten Ausführungsform handelt es sich bei dem Stabilisator um 2,6-Di-tert-butyl-4-methylphenol und/oder 2,6-Di-tert-butyl-phenol.

Vorzugsweise beträgt in dem erfindungsgemäßen Kit der Anteil an Paste A 30 - 70 Gewichtsprozent und der Anteil an Paste B 30 - 70 Gewichtsprozent, bezogen auf das Gesamtgewicht der Pasten A und B.

Erfindungsgemäß dient der Kit, der wenigstens die Pasten A und B enthält, zur Herstellung von Knochenzement.

Dazu werden die wenigstens zwei Pasten A und B miteinander vermischt, wobei eine Knochenzementpaste erhalten wird.

Das Mischungsverhältnis beträgt vorzugsweise 0,5 -1,5 Gewichtsteile an Paste A zu 0,5 - 1,5 Gewichtsteilen an Paste B.

Das Mischen kann mit üblichen Mischgeräten, beispielsweise einem statischen Mischer oder einem dynamischen Mischer, erfolgen.

Das Mischen kann unter Vakuum oder vakuumfrei erfolgen.

Gemäß einer besonders bevorzugten Ausführungsform erfolgt das Mischen der Pasten A und B mit der vorstehend erläuterten Vorrichtung zur Herstellung von Knochenzement. Demnach sind die Pasten A und B getrennt voneinander in zwei Behältern, zum Beispiel einer Doppelkartusche, vorgesehen. Durch das Öffnen wenigstens eines dieser Behälter und vorzugsweise beider Behälter können die in den beiden Behältern enthaltenen Pasten A und B miteinander in Kontakt treten. Ein Vermischen der Pasten A und B kann anschließend durch Betätigen einer Mischeinheit, zum Beispiel eines statischen oder aktiv angetriebenen Mischers, erreicht wer den, die in der Vorrichtung enthalten ist. Zur Applikation kann die Knochenzementpaste schließlich aus der Vorrichtung ausgetragen werden. Diese Applikation der Knochenzementpaste erfolgt vorzugsweise mittels einer Applikationseinheit, die ein Auspressen der Knochenzementpaste ermöglicht.

Nach dem Vermischen der Pasten A und B des Kits ist die letztlich erhaltene Knochenzementpaste nach der Norm ISO 5833 klebfrei und kann sofort verarbeitet werden.

Der aus der Knochenzementpaste durch Aushärtung entstehende Knochenzement erreicht wenige Minuten nach dem Vermischen der im Kit enthaltenen Pasten eine hohe Festigkeit.

Der erfindungsgemäße Kit kann gemäß einer bevorzugten Ausführungsform zur mechanischen Fixierung von Gelenkendoprothesen, zur Abdeckung von Schädeldefekten, zur Auffüllung von Knochenkavitäten, zur Femuroplastie, zur Vertebroplastie, zur Kyphoplastie, zur Herstellung von Spacern und zur Herstellung von Trägermaterialien für die lokale Antibiotika-Therapie verwendet werden.

Unter dem Begriff "Spacer" werden dabei erfindungsgemäß Implantate verstanden, die temporär im Rahmen des zweizeitigen Prothesenwechsels bei septischen Revisionen als Platzhalter verwendet werden.

Trägermaterialien für die lokale Antibiotika-Therapie können als Kugeln oder kugelähnliche Körper oder als bohnenförmige Körper ausgebildet sein. Daneben ist es auch möglich, stabförmige oder scheibenförmige Trägermaterialien herzustellen, die den aus dem erfindungsgemäßen Kit hergestellten Knochenzement enthalten. Weiterhin können die Trägermaterialien auch auf resorbierbarem oder nicht resorbierbarem Fadenmaterial vorzugsweise perlschnurförmig aufgereiht sein.

Die vorstehend beschriebenen erfindungsgemäßen Verwendungen von Knochenzement sind aus der Literatur bekannt und dort mannigfach beschrieben.

Erfindungsgemäß wird der Kit für die vorstehend beschrieben Verwendungen eingesetzt, indem vorzugsweise aus den im Kit enthaltenen Pasten durch Vermischen eine Knochenzementpaste hergestellt wird, die analog zu den aus dem Stand der Technik bekannten Pasten für die beschriebenen Verwendungen eingesetzt wird.

Die Erfindung wird durch die nachstehend beschriebenen Beispiel erläutert, die jedoch die Erfindung nicht einschränken.

### BEISPIELE:

### BEISPIELE 1 - 7 UND VERGLEICHSBEISPIEL 1:

Für die Beispiele 1 - 7 und das Vergleichsbeispiel 1 wurden die Pasten A und B mit einer Zusammensetzung gemäß den nachstehenden Tabellen 1 - 4 hergestellt.

**Tabelle 1: Zusammensetzung der Paste A der Beispiele 1 - 7.**

| **Beispiel Nr.** | **Zusammensetzung der Paste A** | | | | | | |
|---|---|---|---|---|---|---|---|
| | **Radikalisch polymerisierbares Monomere (a1)** | | | **Unlösliches Polymer (a2)** | **Lösliches Polymer (a3)** | **Polymerisationsinitiator 1 (a4)** | **Stabilisator** |
| | **MMA** | **MA** | **EGDMA** | | | | |
| 1 | 20,2 g | - | 0,6 g | 15,5 g | 6,2 g | 2,0 g | 20 mg |
| 2 | 20,2 g | - | 0,6 g | 15,5 g | 6,2 g | 2,0 g | 20 mg |
| 3 | 20,2 g | - | 0,6 g | 15,5 g | 6,2 g | 2,0 g | 20 mg |
| 4 | 20,2 g | - | 0,6 g | 15,5 g | 6,2 g | 2,0 g | 20 mg |
| 5 | 20,2 g | - | 0,6 g | 15,5 g | 6,2 g | 2,0 g | 20 mg |
| 6 | 20,2 g | - | 0,6 g | 15,5 g | 6,2 g | 2,0 g | 20 mg |
| 7 | 20,2 g | - | 0,6 g | 15,5 g | 6,2 g | 1,5 g | 20 mg |

**Tabelle 2: Zusammensetzung der Paste B der Beispiele 1- 7.**

| **Beispiel Nr.** | **Zusammensetzung der Paste B** | | | | | | | |
|---|---|---|---|---|---|---|---|---|
| | **Radikalisch polymerisierbares Monomer (b1)** | | | **Lösliches Polymer (b2)** | **Akzelera tor 1 (b3)** | **Farbstoff** | **Antibiotikum** | **Stabilisator** |
| | **MMA** | **MA** | **EGDMA** | | | | | |
| 1 | 24,2 g | - | - | 16,9 g | 0,4 g | - | - | 35 mg |
| 2 | 20,2 g | - | - | 16,9 g | 0,4 g | - | 3,28 g | 35 mg |
| 3 | 20,2 g | - | - | 16,9 g | 0,4 g | - | 2,19 g | 35 mg |
| 4 | 20,2 g | - | - | 16,9 g | 0,4 g | 50 mg | - | 35 mg |
| 5 | 20,2 g | - | - | 16,9 g | 0,4 g | 50 mg | 2,19 g | 35 mg |
| 6 | 20,2 g | - | - | 16,9 g | 0,4 g | 50 mg | 3,28 g | 36 mg |
| 7 | 20,2 g | - | - | 16,9 g | 0,4 g | 50 mg | - | 35 mg |

**Tabelle 3: Zusammensetzung der Paste A von Vergleichsbeispiel 1.**

| **Vergleichsbeispiel** | **Zusammensetzung der Paste A** | | | | |
|---|---|---|---|---|---|
| | **Radikalisch polymerisierbares Monomer (a1)** | **Unlösliches Polymer (a2)** | **Lösliches Polymer (a3)** | **Polymerisationsinitiator 2 (a4)** | **Stabilisator** |
| 1 | 20,2 g | 15,5 g | 6,3 g | 2,0 g | 20 mg |

**Tabelle 4: Zusammensetzung der Paste B von Vergleichsbeispiel 1.**

| **Vergleichs beispiel** | **Zusammensetzung der Paste B** | | | | | | | |
|---|---|---|---|---|---|---|---|---|
| | **Radikalisch polymerisierbares Monomer (b1)** | **Lösliches Polymer (b2)** | **Unlösliches Polymer (a2)** | **Akzel erator 2 (b3)** | **Chloridsalz** | **Farbstoff** | **Rönt genopaker** | **Stabilisator** |
| 1 | 20,2 g | 11,6 g | 9,8 g | 2 mg | 60 mg | 50 mg | 4,8 g | 35 mg |

Es wurden für die Beispiele 1 - 7 und das Vergleichsbeispiel 1 die folgenden Chemikalien gemäß Tabelle 5 verwendet, die in p.a.-Reinheit vom Chemikaliengroßhandel bezogen wurden:

**Tabelle 5: Bestandteile der Pasten und der Vergleichspaste.**

| **Bestandteil gemäß Tabellen 1 - 4** | **Chemikalie** |
|---|---|
| Radikalisch polymerisierbares Monomer (a1), (a2): | |
| MMA | Methylmethacrylamid |
| MA | Methacrylamid |
| EGDMA | Ethylenglykoldimethacrylat |
| Unlösliches Polymer (a2) | Mit Ethylenglykoldimethacrylat vernetztes Poly(methylmethacrylat) der Siebfraktion < 100 µm. |
| Lösliches Polymer (a3), (b2) | Poly(methylmethacrylat-co-methylacrylat) mit einer Molmasse von < 500000 g/mol |
| Polymerisationsinitiator 1 (a4): | BPO (75%ig) |
| Polymerisationsinitiator 2 (a4): | 1-Cyclohexyh5-ethylbarbiturat |
| Stabilisator | 2,6-Di-t-butyl-4-methyl-phenol |
| Akzelerator 1 (b3) | N,N-2,2-Bis-hydroxyethy\|-p-toluidin (BHET) |
| Akzelerator 2 (b3) | Kupfer(II)-2-ethylhexanoat |
| Farbstoff | Grüner Aluminium-Farblack (Sumrise) |
| Antibiotikum | Gentamicinsulfat (Fujian Fukang Ltd.) mit einem Aktivitätskoeffizienten Alk= 622 |
| Chloridsalz | Aliquat 336 (Trioctylmethylammoniumchlorid) |
| Röntgenopaker | Zirkoniumdioxid |

Zur Herstellung der einzelnen Pasten wurde zunächst jeweils das radikalisch polymerisierbare Monomer (a1) bzw. (a2) und danach der jeweilige Stabilisator in ein inertes Kunststoffgefäß eingewogen. Anschließen wurde zur Herstellung der Paste A der radikalische Polymerisationsinitiator und zur Herstellung der Paste B der Akzelerator in der jeweiligen Vorlage unter Rühren bei Raumtemperatur gelöst. Sodann erfolgte die Zugabe aller weiteren Bestandteile. Die erhaltenen Zubereitungen wurden intensiv miteinander vermischt Es bildeten sich Pasten, die getrennt voneinander bei Raumtemperatur über Nacht gelagert wurden, bis der Endzustand der Quellung erreicht wurde und sich streichfähige Pasten gebildet hatten.

Die Pasten A und B der jeweiligen Beispiele und des Vergleichsbeispiels 1 wurden anschließend miteinander vermischt. Mit den erhaltenen Knochenzementpasten wurden streifenförmige Prüfkörper mit den Abmessungen (75 mm x 10 mm x 3,3 mm) für die Bestimmung der Biegefestigkeit und des Biegemoduls hergestellt und zylindrische Formkörper (Durchmesser 6 mm, Höhe 12 mm) für die Bestimmung der Druckfestigkeit angefertigt. Die Probekörper wurden 24 Stunden bei 23 °C gelagert. Danach wurden die 4-Punkt-Biegefestigkeit, das Biegemodul und die Druckfestigkeit der Prüfkörper mit einer Zwick-Universalprüfmaschine ermittelt.

## Patentansprüche

1. Kit zur Herstellung von Knochenzement wenigstens aufweisend eine Paste A und eine Paste B, wobei
(a) Paste A
(a1) wenigstens ein radikalisch polymerisierbares Monomer,
(a2) wenigstens ein in (a1) unlösliches Polymer,
(a3) wenigstens ein in (a1) lösliches Polymer und
(a4) wenigstens einen radikalischen Polymerisationsinitiator enthält,
wobei das Gewichtsverhältnis von dem wenigstens einen in (a1) unlöslichen Polymer (a2) zu dem wenigstens einen in (a1) löslichen Polymer (a3) wenigstens 2 zu 1 beträgt, und
(b) Paste B
(b1) wenigstens ein radikalisch polymerisierbares Monomer,
(b2) wenigstens ein in (b1) lösliches Polymer und
(b3) wenigstens einen in (b1) löslichen Akzelerator enthält,
(b4) gegebenenfalls ein in (b1) unlösliches Polymer,
wobei der maximale Gehalt an in (b1) unlöslichem Polymer (b4) bei 5 Gewichtsprozent, bezogen auf das Gesamtgewicht von Paste B liegt, und
wobei das Gewichtsverhältnis von in (b1) unlöslichem Polymer (b4) zu dem wenigstens einen in (b1) löslichen Polymer (b2) höchstens 0,2 beträgt.

2. Kit nach einem def Anspruch 1, **dadurch gekennzeichnet, dass** wenigstens eines der radikalisch polymerisierbaren Monomere (a1) und (b1) ein Methacrylatmonomer ist.

3. Kit nach Anspruch 2, **dadurch gekennzeichnet, dass** das wenigstens eine Methacrylatmonomer aus der Gruppe ausgewählt ist, die aus Methylmethacrylat, Ethylenglykoldimethacrylat und Butan-1,4-diol-dimethacrylat besteht.

4. Kit nach einem der Ansprüche 1- 3, **dadurch gekennzeichnet, dass** das in (a1) unlösliche Polymer (a2) ein partikuläres Polymer ist.

5. Kit nach einem der Ansprüche 1 - 4, **dadurch gekennzeichnet, dass** das wenigstens eine in (a1) unlösliche Polymer (a2) aus der Gruppe ausgewählt ist, die aus vernetzten Polymeren besteht.

6. Kit nach einem der Ansprüche 1 - 5, **dadurch gekennzeichnet, dass** wenigstens eines der Polymere (a3) und (b2) aus der Gruppe ausgewählt ist, die aus Poly(methylmethacrylat)-Copolymeren besteht.

7. Kit nach Anspruch 6, **dadurch gekennzeichnet, dass** das Poly(methylmethacrylat)-Copolymer aus der Gruppe ausgewählt ist, die aus Poly(methylmethacrylat-co-methylacrylat) und Poly(methylmethacrylat-co-styrol) besteht.

8. Kit nach einem der Ansprüche 1- 7, **dadurch gekennzeichnet, dass** in Paste B kein unlösliches Polymer (b4) enthalten ist.

9. Kit nach einem der Ansprüche 1- 8, **dadurch gekennzeichnet, dass** der wenigstens eine radikalische Polymerisationsinitiator (a4) aus der Gruppe ausgewählt ist, bestehend aus (i) Peroxiden und (ii) Barbituraten, die aus der Gruppe ausgewählt sind, die aus 1,5-Dialkyl-baribituraten, 1-Cycloalkyl-5-alkyl-barbituraten, 1-Aryl-5-alkyl-barblturaten besteht.

10. Kit nach einem der Ansprüche 1- 9, **dadurch gekennzeichnet, dass** der wenigstens eine in (b1) lösliche Polymerisationsakzelerator (b3) aus der Gruppe ausgewählt ist, die aus N,N-Dimethyl-p-toluidin, N,N-Bis-hydroxyethyl-p-toluidin, N,N-Dimethyl-anilin, 4-N,N-Dimethylamino-pyridin, Saccharin, Lithiumchlorid, Trioctylmethylammoniumchlorid, organischen Kupfer(II)-salzen und Mischungen davon besteht.

11. Kit nach einem der Ansprüche 1-10. **dadurch gekennzeichnet, dass**
Paste A 15 -75 Gewichtsprozent des wenigstens einen radikalisch polymerisierbaren Monomers (a1), 20 - 70 Gewichtsprozent des wenigstens einen in (a1) unlöslichen Füllstoffs (a2), 1- 25 Gewichtsprozent des wenigstens einen in (a1) löslichen Polymers (a3) und 0,00001 -15 Gewichtsprozent des wenigstens einen radikalischen Polymerisationsinitiators (a4), bezogen auf das Gesamtgewicht von Paste A, enthält, und
Paste B10 - 70 Gewichtsprozent des wenigstens einen radikalisch polymerisierbaren Monomers (b1), 25 - 85 Gewichtsprozent des wenigstens einen in (b1) löslichen Polymers (b2) und 0,00001 -15 Gewichtsprozent des wenigstens einen Akzelerators (b3), bezogen auf das Gesamtgewicht von Paste B, enthält.

12. Kit nach einem der Ansprüche 1-11, **dadurch gekennzeichnet, dass** wenigstens eine der Pasten A und B einen Röntgenopaker enthält.

13. Kit nach einem der Ansprüche 1-12, **dadurch gekennzeichnet, dass** wenigstens eine der Pasten A und B wenigstens einen pharmazeutischen Wirkstoff enthält.

14. Kit nach einem der Ansprüche 1-13, **dadurch gekennzeichnet, dass** der Kit als Vorrichtung zur Herstellung von Knochenzement ausgestaltet ist, die
(i) einen ersten Behälter und einen zweiten Behälter aufweist, wobei der erste Behälter die Paste A und der zweite Behälter die Paste B aufweist, wobei wenigstens einer der Behälter offenbar ist, um nach dem Öffnen ein Vermischen der Pasten A und B zu gestatten, und
(ii) eine Mischeinheit zum Vermischen der Pasten A und B.

15. Verfahren zum Herstellen von Knochenzement, bei dem
(i) ein Kit zur Herstellung von Knochenzement wenigstens aufweisend eine Paste A und eine Paste B, wobei
(a) Paste A
(a1) wenigstens ein radikalisch polymerisierbares Monomer,
(a2) wenigstens ein in (a1) unlösliches Polymer,
(a3) wenigstens ein in (a1) lösliches Polymer und
(a4) wenigstens einen radikalischen Polymerisationsinitiator enthält,
wobei das Gewichtsverhältnis von dem wenigstens einen in (a1) unlöslichen Polymer (a2) zu dem wenigstens einen in (a1) löslichen Polymer (a3) wenigstens 2 zu 1 beträgt, und
(b) Paste B
(b1) wenigstens ein radikalisch polymerisierbares Monomer,
(b2) wenigstens ein in (b1) lösliches Polymer und
(b3) wenigstens einen in (b1) löslichen Akzelerator enthält,
(b4) gegebenenfalls ein in (b1) unlösliches Polymer,
wobei der maximale Gehalt an in (b1) unlöslichem Polymer (b4) bei 5 Gewichtsprozent, bezogen auf das Gesamtgewicht von Paste B liegt, und wobei das Gewichtsverhältnis von in (b1) unlöslichem Polymer (b4) zu dem wenigstens einen in (b1) löslichen Polymer (b2) höchstens 0,2 beträgt, bereitgestellt wird und
(ii) die Pasten A und B miteinander vermischt werden.

## Claims

1. Kit for the production of bone cement at least comprising one paste A and one paste B, whereby
(a) paste A contains
(a1) at least one monomer for radical polymerisation;
(a2) at least one polymer that is insoluble in (a1);
(a3) at least one polymer that is soluble in (a1); and
(a4) at least one radical polymerisation initiator,
whereby the weight ratio of the at least one polymer (a2) that is insoluble in (a1) and the at least one polymer (a3) that is soluble in (a1) is at least 2 to 1; and
(b) paste B contains
(b1) at least one monomer for radical polymerisation;
(b2) at least one polymer that is soluble in (b1); and
(b3) at least one accelerator that is soluble in (b1);
(b4) possibly a polymer that is insoluble in (b1);
whereby the maximal content of polymer (b4) that is insoluble in (b1) is 5% by weight, relative to the total weight of paste B, and
whereby the weight ratio of polymer (b4) that is insoluble in (b1) and the at least one polymer (b2) that is soluble in (b1) is at most 0.2.

2. Kit according to claim 1, **characterised in that** at least one of the monomers (a1) and (b1) for radical polymerisation is a methacrylate monomer.

3. Kit according to claim 2, **characterised in that** the at least one methacrylate monomer is selected from the group consisting of methylmethacrylate, ethylene glycol dimethacrylate, and butane-1,4-diol-dimethacrylate.

4. Kit according to any one of the claims 1 - 3, **characterised in that** the polymer (a2) that is insoluble in (a1) is a particulate polymer.

5. Kit according to any one of the claims 1 - 4, **characterised in that** the at least one polymer (a2) that is insoluble in (a1) is selected from the group consisting of cross-linked polymers.

6. Kit according to any one of the claims 1 - 5, **characterised in that** at least one of the polymers (a3) and (b2) is selected from the group consisting of poly(methylmethacrylate) copolymers.

7. Kit according to claim 6, **characterised in that** the poly(methylmethacrylate) copolymer is selected from the group consisting of poly(methylmethacrylate-co-methylacrylate) and poly(methylmethacrylate-co-styrene).

8. Kit according to any one of the claims 1 - 7, **characterised in that** paste B contains no insoluble polymer (b4).

9. Kit according to any one of the claims 1 - 8, **characterised in that** the at least one radical polymerisation initiator (a4) is selected from the group consisting of (i) peroxides and (ii) barbiturates selected from the group consisting of 1,5-dialkyl-barbiturates, 1-cycloalkyl-5-alkyl-barbiturates, 1-aryl-5-alkylbarbiturates.

10. Kit according to any one of the claims 1 - 9, **characterised in that** the at least one polymerisation accelerator (b3) that is soluble in (b1) is selected from the group consisting of N,N-dimethyl-p-toluidine, N,N-bis-hydroxyethyl-p-toluidine, N,N-dimethylaniline, 4-N,N-dimethylamino-pyridine, saccharine, lithium chloride, trioctylmethylammoniumchloride, organic copper(II) salts, and mixtures thereof.

11. Kit according to any one of the claims 1 - 10, **characterised in that**
paste A contains 15 - 75% by weight of the at least one monomer for radical polymerisation (a1), 20 - 70% by weight of the filling agent (a2) that is insoluble in (a1), 1 - 25% by weight of the at least one polymer (a3) that is soluble in (a1), and 0.00001 - 15% by weight of the at least one radical polymerisation initiator (a4), relative to the total weight of paste A, and
paste B contains 10 - 70% by weight of the at least one monomer for radical polymerisation (b1), 25 - 85% by weight of the at least one polymer (b2) that is soluble in (b1), and 0.00001 - 15% by weight of the at least one accelerator (b3), relative to the total weight of paste B.

12. Kit according to any one of the claims 1 - 11, **characterised in that** at least one of the pastes A and B contains a radiopaquer.

13. Kit according to any one of the claims 1 - 12, **characterised in that** at least one of the pastes A and B contains at least one pharmaceutical agent.

14. Kit according to any one of the claims 1 - 13, **characterised in that** the kit is designed as a device for the production of bone cement, comprising
(i) a first container and a second container, whereby the first container comprises paste A and the second container comprises paste B, whereby at least one of the containers can be opened to allow for mixing of pastes A and B after the opening, and
(ii) a mixing unit for mixing pastes A and B.

15. Method for the production of bone cement, in which
(i) a kit for the production of bone cement at least comprising a paste A and a paste B is provided, whereby
(a) paste A contains
(a1) at least one monomer for radical polymerisation;
(a2) at least one polymer that is insoluble in (a1);
(a3) at least one polymer that is soluble in (a1); and
(a4) at least one radical polymerisation initiator,
whereby the weight ratio of the at least one polymer (a2) that is insoluble in (a1) and the at least one polymer (a3) that is soluble in (a1) is at least 2 to 1; and
(b) paste B contains
(b1) at least one monomer for radical polymerisation;
(b2) at least one polymer that is soluble in (b1); and
(b3) at least one accelerator that is soluble in (b1);
(b4) possibly a polymer that is insoluble in (b1);
whereby the maximal content of polymer (b4) that is insoluble in (b1) is 5% by weight, relative to the total weight of paste B, and
whereby the weight ratio of polymer (b4) that is insoluble in (b1) and the at least one polymer (b2) that is soluble in (b1) is at most 0.2; and
(ii) pastes A and B are being mixed with each other.

## Revendications

1. Kit pour la fabrication de ciment osseux présentant au moins une pâte A et une pâte B, dans lequel
(a) la pâte A
(a1) contient au moins un monomère polymérisable par voie radicalaire,
(a2) au moins un polymère insoluble dans (a1),
(a3) au moins un polymère soluble dans (a1) et
(a4) au moins un initiateur de polymérisation radicalaire,
dans lequel le rapport pondéral de l'au moins un polymère (a2) insoluble dans (a1) sur l'au moins un polymère (a3) soluble dans (a1) se monte à au moins 2 sur 1, et
(b) la pâte B
(b1) contient au moins un monomère polymérisable par voie radicalaire,
(b2) au moins un polymère soluble dans (b1) et
(b3) au moins un accélérateur soluble dans (b1),
(b4) éventuellement un polymère insoluble dans (b1),
dans lequel la teneur maximale en polymère (b4) insoluble dans (b1) se situe à 5 pourcent en poids par rapport au poids total de la pâte B, et
dans lequel le rapport pondéral du polymère (b4) insoluble dans (b1) sur l'au moins un polymère (b2) soluble dans (b1) se monte à 0,2 maximum.

2. Kit selon la revendication 1, **caractérisé en ce qu'**au moins un des monomères (a1) et (b1) polymérisables par voie radicalaire est un monomère de méthacrylate.

3. Kit selon la revendication 2, **caractérisé en ce que** l'au moins un monomère de méthacrylate est sélectionné parmi le groupe qui se compose du méthacrylate de méthyle, du diméthacrylate d'éthylèneglycol et du butane-1,4-diol-diméthacrylate.

4. Kit selon une des revendications 1 à 3, **caractérisé en ce que** le polymère (a2) insoluble dans (a1) est un polymère particulaire.

5. Kit selon une des revendications 1 à 4, **caractérisé en ce que** l'au moins un polymère (a2) insoluble dans (a1) est sélectionné parmi le groupe qui se compose de polymères réticulés.

6. Kit selon une des revendications 1 à 5, **caractérisé en ce qu'**au moins un des polymères (a3) et (b2) est sélectionné parmi le groupe qui se compose de copolymères de poly(méthylméthacrylate).

7. Kit selon la revendication 6, **caractérisé en ce que** le copolymère de poly(méthylméthacrylate) est sélectionné parmi le groupe qui se compose du poly(méthylméthacrylate-co-méthylacrylate) et du poly(méthylméthacrylate-co-styrène).

8. Kit selon une des revendications 1 à 7, **caractérisé en ce qu'**aucun polymère insoluble (b4) n'est contenu dans la pâte B.

9. Kit selon une des revendications 1 à 8, **caractérisé en ce que** l'au moins un initiateur de polymérisation radicalaire (a4) est sélectionné parmi le groupe constitué de (i) peroxydes et (ii) barbituriques qui sont sélectionnés parmi le groupe qui se compose des 1,5-dialkyl-barbituriques, 1-cycloalkyl-5-alkyl-barbituriques, 1-aryl-5-alkyl-barbituriques.

10. Kit selon une des revendications 1 à 9, **caractérisé en ce que** l'au moins un accélérateur de polymérisation (b3) soluble dans (b1) est sélectionné parmi le groupe qui se compose de la N,N-diméthyl-p-toluidine, N,N-bis-hydroxyéthyl-p-toluidine, N,N-diméthyl-aniline, 4-N,N-diméthylamino-pyridine, saccharine, du chlorure de lithium, chlorure de trioctylméthylammonium, des sels de cuivre(II) organiques et de mélanges de ceux-ci.

11. Kit selon une des revendications 1 à 10, **caractérisé en ce que**
la pâte A contient 15 à 75 pourcent en poids de l'au moins un monomère polymérisable par voie radicalaire (a1), 20 à 70 pourcent en poids de l'au moins une charge (a2) insoluble dans (a1), 1 à 25 pourcent en poids de l'au moins un polymère (a3) soluble dans (a1) et 0,00001 à 15 pourcent en poids de l'au moins un initiateur de polymérisation radicalaire (a4), par rapport au poids total de la pâte A, et
la pâte B contient 10 à 70 pourcent en poids de l'au moins un monomère polymérisable par voie radicalaire (b1), 25 à 85 pourcent en poids de l'au moins un polymère (b2) soluble dans (b1) et 0,00001 à 15 pourcent en poids de l'au moins un accélérateur (b3), par rapport au poids total de la pâte B.

12. Kit selon une des revendications 1 à 11, **caractérisé en ce qu'**au moins une des pâtes A et B contient un opacifiant radiographique.

13. Kit selon une des revendications 1 à 12, **caractérisé en ce qu'**au moins une des pâtes A et B contient au moins un ingrédient actif pharmaceutique.

14. Kit selon une des revendications 1 à 13, **caractérisé en ce que** le kit est configuré comme dispositif pour la fabrication de ciment osseux qui
(i) présente un premier récipient et un second récipient, dans lequel le premier récipient présente la pâte A et le second récipient présente la pâte B, dans lequel au moins un des récipients peut être ouvert pour permettre après l'ouverture un mélange des pâtes A et B, et
(ii) un module de mélange pour le mélange des pâtes A et B.

15. Procédé pour la fabrication de ciment osseux, lors duquel
(i) un kit pour la fabrication de ciment osseux présentant au moins une pâte A et une pâte B est mis à disposition, dans lequel
(a) la pâte A
(a1) contient au moins un monomère polymérisable par voie radicalaire,
(a2) au moins un polymère insoluble dans (a1),
(a3) au moins un polymère soluble dans (a1) et
(a4) au moins un initiateur de polymérisation radicalaire,
dans lequel le rapport pondéral de l'au moins un polymère (a2) insoluble dans (a1) sur l'au moins un polymère (a3) soluble dans (a1) se monte à au moins 2 sur 1, et
(b) la pâte B
(b1) contient au moins un monomère polymérisable par voie radicalaire,
(b2) au moins un polymère soluble dans (b1) et
(b3) au moins un accélérateur soluble dans (b1),
(b4) éventuellement un polymère insoluble dans (b1),
dans lequel la teneur maximale en polymère (b4) insoluble dans (b1) se situe à 5 pourcent en poids par rapport au poids total de la pâte B, et
dans lequel le rapport pondéral du polymère (b4) insoluble dans (b1) sur l'au moins un polymère (b2) soluble dans (b1) se monte à 0,2 maximum, et
(ii) les pâtes A et B sont mélangées l'une avec l'autre.
